# EUROPEAN PATENT APPLICATION

(11) **EP 2 544 039 A1**
(43) Date of publication of application: **09.01.2013**
(21) Application number: 11250633.2
(22) Date of filing: 04.07.2011
(51) Int. Cl.: G02B 27/01, G06F 3/01, A61B 19/00, G02B 27/00

(54) **Display system**

(71) Applicant: BAE Systems Plc., London SW1Y 5AD (GB)
(72) Inventor: The designation of the inventor has not yet been filed
(74) Representative: BAE SYSTEMS plc Group IP Department

(57) **Abstract**

A display system for displaying a virtual image to a viewer superimposed over a real object. The virtual image is conformal, meaning that the virtual image conforms to the real object along the line of sight of the viewer during movement of the viewer relative to the object. If a viewer changes his line of sight, the virtual image changes to conform to the real object along the changed line of sight The display system comprises a camera adapted to be mounted to the head of a human viewer and display apparatus adapted to be placed in front of at least one eye of the human viewer, the display apparatus being physically connected to, and in a fixed spatial relationship with, the camera. A method of displaying conformal virtual images using this apparatus is also described.

## Description

This invention relates to a display system for displaying a virtual image to a viewer superimposed over a real object or scene.

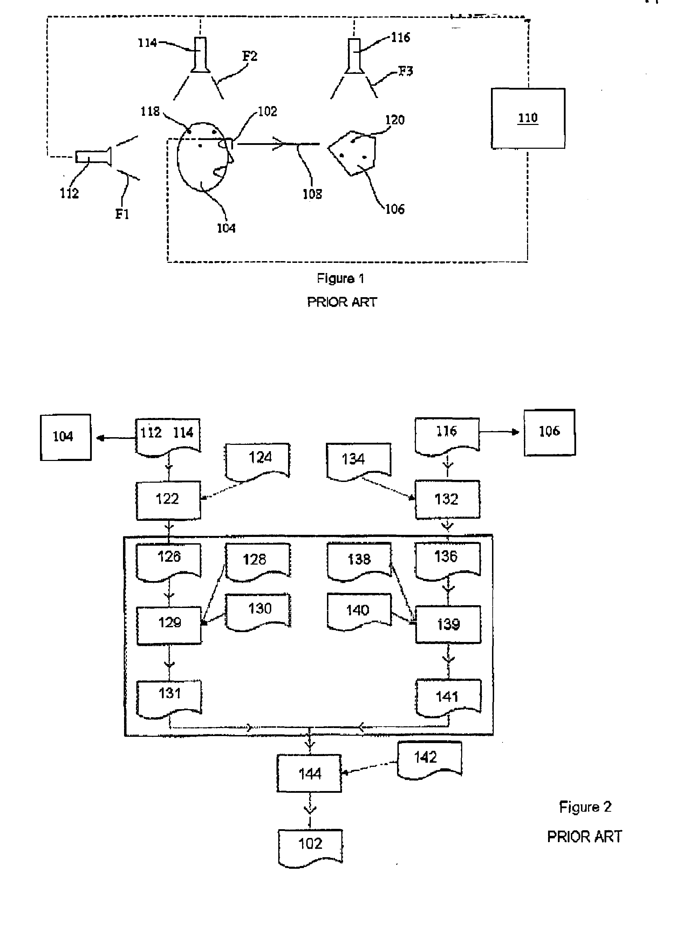

Figure 1 shows a prior art display system 100. A display apparatus 102 which functions as a combiner shows a virtual image to a viewer 104 superimposed over a real object 106. The virtual image displayed is conformal, meaning that the virtual image conforms to the real object along a line of sight 108 of the viewer during movement of the viewer relative to the object. That is, if a viewer changes his line of sight, the virtual image changes to conform to the real object along the changed line of sight For example, in one application of the display system, the viewer 104 may be a surgeon and the object 106 may be a patient. The virtual image may be an X-ray or MRI scan of the patient. If the surgeon's line of sight is coincident with a particular part of the patient's head the display shows the X-ray or MRI scan of that part of the patient's head to help the surgeon operate on the patient.

In order to make the virtual image conformal, the display system senses, or tracks, the position and orientation of the viewer 104 and the position and orientation of the object 106, and depending on the tracked positions, a control 110 determines the virtual image to be displayed on the display apparatus 102 for conforming with the object along the line of sight. Cameras 112, 114 have fields of view F1 and F2 and, provided the viewer 104 is within the field of view of cameras 112, 114, the position and orientation of the viewer 104 relative to the cameras 112, 114 can be determined. Typically, the cameras 112, 114 have fixed locations which are stored in the control and therefore the absolute location and orientation of the viewer may be determined. Similarly, a fixed camera 116 has a field of view F3 within which the absolute location and orientation of the object 106 may be determined.

The fields of view F1, F2 and F3 are finite and therefore in order to expand the range of positions in which the location of the viewer and object may be determined, the cameras may be mounted to their fixed locations in a manner which allows pivotal movement. In this case, the orientation of the cameras must be determined and taken into account when calculating the position and orientation of the viewer 104 and of the object 106. Furthermore, the cameras may have variable focal lengths in order to zoom into and out from the viewer and object. Accordingly, the focal length of each camera must additionally be determined and used in calculating the orientation and position of the viewer and of the object.

In this example, the viewer and the object are collaborative in the sense that both the viewer and the object wish their locations to be readily determined. In this regard, a locating device or devices 118 are fixed relative to the viewer 104. The locating devices are configured to be readily sensed by the cameras 112, 114 and may for example be light emitting diodes (LEDs) or other prominently visible devices. Likewise, a locating device or devices 120 are fixed relative to the object and are configured to be readily sensed by the camera 116. In the arrangement shown, three locating devices are fixed relative to each of the viewer and the object to allow triangulation of their locations. In this way, movement and orientation can be determined in all possible degrees of freedom.

Although it is possible for the display system to function if the viewer and object are not collaborative, it requires the use of complex recognition algorithms and processing, which decreases accuracy and/or increases cost

A flow diagram indicating the functionality of the display system is shown in Figure 2. There are two determination paths which are combined in the display control for displaying the correct resultant virtual image on the display apparatus. The first path constitutes determination of the viewer's absolute position and orientation, whilst the second path constitutes the object's absolute position and orientation.

Referring to Figure 2, cameras 112, 114 are directed towards the viewer 104, and an image of the viewer is generated and conveyed for processing by image processor 122. The focal length of cameras 112, 114 is also established, and this camera zoom variable 124 is fed to image processor 122. Thus the camera zoom variable 124 is taken into account and used by the image processor 122 in calculating the position and orientation of the viewer 104. The image processor 122 must carry out several steps in order to determine the position and orientation of the viewer 104. After receiving the camera image of the viewer and the camera zoom variable data, the image processor calculates the position of the viewer's head relative to the cameras 112, 114 (step 126 in Figure 2). The data 128 concerning orientation of cameras 112, 114 and data 130 concerning position of cameras 112, 114 is fed into the image processor 122, and this data 128, 130 is used in conjunction with the data obtained in step 126 to allow the head of the viewer 104 to be tracked 129 as the viewer moves or rotates his head.

Similarly, camera 116 is directed towards the object 106, and an image of the object is generated and conveyed for processing by image processor 132. The focal length of the camera 116 is also established, and this camera zoom variable 134 is fed to image processor 132. Thus the camera zoom variable 134 is taken into account and used by the image processor 132 in calculating the position and orientation of the object being tracked 106. The image processor 132 must carry out several steps in order to determine the position and orientation of the object being tracked 106. After receiving the camera image of the object 106 and the camera zoom variable data 134, the image processor 132 calculates the position of the tracked object 106 relative to the camera 116 (step 136 in Figure 2). The data 138 concerning orientation of the camera 116 and data 140 concerning the position of the camera 116 is fed into the image processor 132, and this data 138, 140 is used in conjunction with the data obtained in step 136 to allow the object to be tracked 139 as it moves.

It can be seen that the prior art requires two different camera imaging and tracking systems, one for the viewer and one for the object. To allow a conformal virtual image to be displayed to the viewer 104 on the display apparatus 102, data from the two different camera systems needs to be combined. To allow this, the absolute position 131 of the viewer's head 104 must be established relative to a fixed x=0, y=a, z=0 (0,0,0) point. Similarly, the absolute position 141 of the object 106 must be established relative to the same (0,0,0) point. This information, along with viewer field of view data 142, is fed to the display control system 144. The display control system processes the incoming information and uses it to generate a virtual image which is displayed on the display apparatus 102. The display apparatus 102 permits the viewer to see the real object through the display apparatus, but also superimposes a virtual conformal image over the real one as seen by the viewer.

A problem with the prior art is that each processing step introduces a small error and the number of processing steps required in the Figure 1 arrangement means that the virtual image may not conform exactly to the real object as viewed by the viewer. This is due to accumulated errors within the prior art systems.

The present invention seeks to provide an improved display system, by offering a solution with fewer accumulated errors than in known display systems.

Accordingly the present invention provides a display system for displaying to a viewer a conformal image overlaying an object along a line of sight of the viewer, the display system comprising a sensor adapted to be mounted to the head of a human viewer for sensing the position and orientation of the object relative to the viewer and display apparatus adapted to be placed in front of at least one eye of the human viewer for displaying to the viewer an image conforming to the sensed position and orientation of the object, the display apparatus being located in a fixed spatial relationship with the sensor.

This arrangement results in a display system with fewer accumulated errors than the prior art, because it is not necessary to track both the viewer and the object, determine the absolute position and orientation of the viewer and of the object, and then determine the relative position and orientation of the object with respect to the viewer, all of which contain errors which accumulate with every step in the process. Instead, the present invention provides a display system which does not require that the viewer be tracked, but rather that the object is tracked directly by the camera located on the viewer's head. This simplifies the calculation of, and reduces the error in, the object position with respect to the viewer. Furthermore, the fixed physical spacing between the camera and the display apparatus means that the position of one relative to the other is constant, resulting in the elimination of another variable, and hence a reduction in error of the overall system.

The sensor may be configured to output a signal corresponding to the position and orientation of the object and the system further comprises a control for receiving said signal and determining the position and orientation of the object relative to the viewer.

The control may be configured to output a signal to the display apparatus for controlling the display of the conformal image dependent on the determined position and orientation of the object and the fixed spatial relationship between the sensor and the display apparatus.

The sensor may be a camera configured for capturing an image of the object.

Advantageously, the camera may be provided with a zoom function to allow the camera to zoom into and out from the object. Where a zoom function is provided, the focal length of the camera must additionally be determined and used in calculating the position of the object relative to the viewer. An image processor is preferably provided to allow the camera image to be processed, for example to allow a camera having a variable focal length to be taken into account.

The display apparatus may be incorporated into or fixed onto a helmed or other headgear worn by the viewer. The camera may also be incorporated into or fixed onto the helmet or other headgear. The display apparatus may be incorporated into spectacles, goggles or other eyewear worn by the viewer. Alternatively, the display apparatus may be incorporated into an eyepiece adapted to be located In front of one of the viewer eyes.

The conformal image may include graphics such as cross-hairs, alphanumeric, lines to aid measurement, or other graphics.

The display system may also comprise a memory store for storing data relating to the object and the control is configured for selective retrieval of said data for processing said data depending on at least the sensed position and orientation of the object and controlling the display apparatus in accordance with the processed data to display the conformal image. The memory store may Contain, for example, images of a previously undertaken MRI scan of a patient, or design data relating to aircraft, ships, tanks, missiles or other objects. The images contained in the memory store are advantageously superimposed over the image of the real object as seen by the viewer, to allow the viewer to see details of the object not ordinarily visible, for example details of the internal structure of the object. By superimposing of virtual image of for example, an earlier MRI scan of the patient (the object) over the correct position within the surgeon's field of view, the surgeon may perform his operation more quickly and accurately.

When the object is not within the field of view of the viewer, the display system may be adapted to switch off the superimposed virtual image so that the viewer is not distracted.

In situations where the object and the viewer are collaborative in the sense that the object wishes to be tracked by the viewer, locating devices may be fixed on the object to allow the object to readily sensed by the camera. LEDs or other prominently visible devices may be used as locating devices.

In situations where the object and viewer are not collaborative, the camera may be used to sense the object as long as it is within the field of view of the camera.

Where the object is not in the field of view of the camera, a second camera may be used to locate the object. Once the object is within the field of view of the first camera, the second camera may be switched off, and the display system will function as previously described.

The invention further provides a method of displaying to a viewer a conformal image overlaying an object along a line of sight of the viewer, the method comprising mounting a sensor to a viewer, sensing the position and orientation of the object relative to the viewer, displaying to the viewer on a display apparatus in fixed spatial relationship with the sensor an image conforming to the sensed position and orientation off the object.

The present invention further comprises a method of displaying information comprising the steps of: providing display system comprising a sensor and a display apparatus, the display apparatus being physically connected to, and in a fixed spatial relationship with, the camera, mounting the display system to the head of a human viewer; locating the display apparatus in front of at least one eye of the human viewer, directing the sensor towards an object by head movement of the human viewer; using the sensor to sense a position and orientation of the object relative to the viewer, generating a conformal image dependent on the sensed position and orientation of the abject; and displaying the conformal image on the display apparatus such that the image is superimposed over, and conforms to, the real object as seen by the human viewer.

The invention will now be described by way of example only with reference to the accompanying drawings wherein:
Figures 1 and 2 show a prior art display system, as described earlier in this patent specification.
Figures 3 and 4 show an embodiment of the present invention.

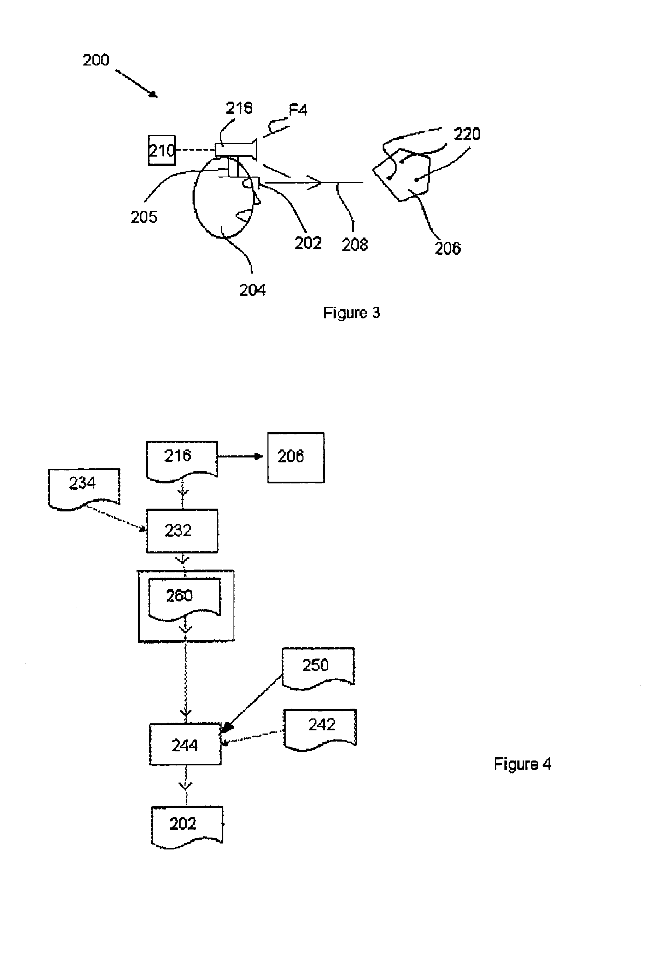

Figure 3 shows a display system 200 in accordance with one arrangement of the present invention. A display apparatus 202 which functions as an image combiner shows a virtual image superimposed over a real object 206 as seen by a viewer 204. The viewer 204 is able to look through the display apparatus 202 at the object 206, and simultaneously sees a conformal virtual image displayed on display apparatus 202. Conformal means that the virtual image conforms to the image that the viewer sees of the real object along a line of sight 208 of the viewer 204 during movement of the viewer relative to the object 206. This means that if the viewer changes their line of sight the virtual image displayed on the display apparatus 202 changes to conform to the image of the object as seen by the viewer along the new line of sight

In this example, the viewer 204 is a surgeon and the object 206 is a patient The surgeon 204 is equipped with a head mounted camera 216 and display apparatus 202. The camera 216 and display apparatus 202 are connected to each other and are held in a fixed spatial relationship with respect to each other for example by one or more rigid struts 205. The struts 205 may be made of metal, plastic, or other suitable material that does not readily stretch or deform at typical temperature. The camera may be mounted on the surgeon's head by means of an elastic headband, or a rigid headband, or may alternatively be located on, or incorporated into, a hat, helmet or other headgear worn by the surgeon.

As the surgeon looks at the patient, the camera 216 senses the position and orientation of the patient with respect to the camera 216 (and therefore the surgeon's head, on which the camera 216 is mounted).

Three locating devices 220 are fixed to the patient to allow the location of the patient to be readily sensed by the camera 216. The locating devices may be LEDs or other prominently visible devices. It should be noted that the locating devices are an optional feature designed to make the patient more visible to the camera.

Whilst a camera has been described as forming part of the display system, the camera may be replaced by any sensor for sensing the position of the object. For example, the sensor may be configured to locate the positions and orientation of the object by sensing the positions of the locating devices. It Is not essential to the invention that the sensor may also be configured for capturing an image of the object.

The camera 216 may optionally have a variable focal length to allow zooming into and out from the patient Where a camera has a zoom capability, the focal length of the camera must be determined and used in calculating the position and orientation of the patient with respect to the surgeon.

A control 210 determines the virtual image to be displayed on display apparatus 202, in accordance with the calculated position and orientation of the patient with respect to the surgeon, such that the virtual image conforms to the surgeon's view of the patient. To aid the surgeon in operating on the patient, the control 210 can access a memory store which contains prior saved data relating to the patient. The memory may be integral with the control or remote from the control and connected to the control by a data link or network. For example, the data may be an X-ray image or an MRI scan of that part of the patient on which the surgeon is operating. As the surgeon looks at a particular part of the patient's body, the display apparatus 202 shows a virtual image of the X-ray or MRI scan of that part of the patient superimposed over the surgeon's view of the patient. This allows the surgeon to effectively see what the internal state of the patient is, and direct his treatment accordingly. In one application, the surgeon may be performing key-hole surgery through a confined aperture in the patient and the display may produce a conformal image of the interior of the patient which would otherwise not be visible. It will be appreciated that the present invention can be used to improve the accuracy of the surgeon's incisions, and also reduce damage done to neighbouring healthy areas of the patient.

A flow diagram showing the functionality of this display system is shown in Figure 4. Referring to Figure 4, a camera 216 which is mounted on the head of the viewer 204 is directed towards the object 206. The camera 216 generates an image of the object 206 and this is processed by an image processor 232 to determine the positional and orientational data 260 of the object with respect to the camera 216 (and hence the viewer). For cameras having a zoom function, the focal length of the camera 216 is also established and this camera zoom variable 234 is fed to the image processor 232. Thus the camera zoom variable 232 is taken into account and used by the image processor 232 In calculating the positional and orientational data 260 of the object 206 with respect to the viewer 204. This data 260, along with the viewer field of view data 242 and the data 250 concerning the position of the camera relative to the display apparatus (a constant as the camera and the display apparatus are held in a fixed spatial relationship with respect to each other) is fed to the display control system 244. The display control system 244 processes the received data 260, 242, 250 and uses it to generate a virtual image which is displayed on the display apparatus 202. The display apparatus 202 permits the viewer to see the real object through the display apparatus, but also superimposes a virtual conformal image over the real one as seen by the viewer.

In this example, a surgeon and a patient have been described. The invention is equally applicable to other situations, such as a fighter pilot (the viewer) and a friendly aircraft (the object), or a pilot (the viewer) and an aircraft carrier with landing strip (the object). The invention may also be used, for example, in carrying out repair or maintenance work on vehicles, structures, or other objects. In such cases the virtual image may be design data relating to the internal structure of the vehicle, structure or other object or may be previously obtained images of the object, for example X-rays or other scans which show the internal condition of the object and can be used to indicate where repair work needs to be carried out.

The image processor and the system control may be located on the viewer. Alternatively one or both may be located remote from the viewer and in this case signals may be transmitted from the remotely located control system to the display apparatus 202.

The virtual image Itself is generated and displayed on the display apparatus using techniques already known in the art. For example, WO2010125378 teaches how a virtual image might be generated and displayed on display apparatus.

## Claims

1. A display system for displaying to a viewer a conformal image overlaying an object along a line of sight of the viewer, the display system comprising a sensor adapted to be mounted to the head of a human viewer for sensing the position and orientation of the object relative to the viewer and display apparatus adapted to be placed in front of at least one eye of the human viewer for displaying to the viewer an image conforming to the sensed position and orientation of the object, the display apparatus being located in a fixed spatial relationship with the sensor.

2. A display system as claimed in Claim 1 wherein the sensor is configured to output a signal corresponding to the position and orientation of the object and the system further comprises a control for receiving said signal and determining the position and orientation of the object relative to the viewer.

3. A display system as claimed in Claim 2, wherein the control is configured to output a signal to the display apparatus for controlling the display of the conformal image dependent on the determined position and orientation of the object and the fixed spatial relationship between the sensor and the display apparatus.

4. A display system as claimed in any of the preceding claims, wherein the sensor is a camera configured for capturing an image of the object.

5. A display system as claimed in Claim 4, wherein the camera has a variable focal length and is configured for outputting a zoom signal corresponding to the focal length, and the control is configured for receiving said zoom signal and for controlling the display of the conformal image dependent on the zoom signal.

6. A display system as claimed in any of the preceding claims, wherein the camera and the display apparatus are incorporated into or fixed onto a helmet or other headgear worn by the human viewer.

7. A display system as claimed in any of the preceding claims, comprising a memory store for storing data relating to the object and the control is configured for selective retrieval of said data for processing said data depending on at least the sensed position and orientation of the object and controlling the display apparatus in accordance with the processed data to display the conformal image.

8. A display system as claimed in claim 5 wherein at least one locating device is positioned on the object and the sensor is configured for sensing the locating devices to allow the object to be readily sensed by the sensor.

9. A display system as claimed in any of claims 5 to 7 wherein the display system is adapted to switch off the conformal virtual image when the object is not within the field of view of the viewer.

10. A display system as claimed in any of claims 5 to 7 wherein the display system comprises a second sensor for sensing the object when the object is not within the field of view of the first sensor.

11. A method of displaying to a viewer a conformal image overlaying an object along a line of sight of the viewer, the method comprising mounting a sensor to a viewer, sensing the position and orientation of the object relative to the viewer, displaying to the viewer on a display apparatus in fixed spatial relationship with the sensor an image conforming to the sensed position and orientation of the object.

12. A method of displaying information comprising the steps of:
providing display system comprising a sensor and a display apparatus, the display apparatus being physically connected to, and in a fixed spatial relationship with, the camera;
mounting the display system to the head of a human viewer,
locating the display apparatus in front of at least one eye of the human viewer,
directing the sensor towards an object by head movement of the human viewer,
using the sensor to sense a position and orientation of the object relative to the viewer;
generating a conformal image dependent on the sensed position and orientation of the object; and
displaying the conformal image on the display apparatus such that the image is superimposed over, and conforms to, the real object as seen by the human viewer.
